# FASCICULE DE BREVET EUROPEEN

(11) **EP 1 032 447 B1**
(45) Date de publication et mention de la délivrance du brevet: **15.09.2004**
(21) Numéro de dépôt: 98955737.6
(22) Date de dépôt: 25.11.1998
(51) Int. Cl.: A61M 11/06

(54) **EMBOUT DE PULVERISATEUR AVEC MEMBRANE DE FERMETURE**
SPRÜHKOPF MIT VERSCHLUSSMEMBRAN
SPRAYER NOZZLE WITH CLOSING MEMBRANE

(30) Priorité: 25.11.1997 FR 9714762
(43) Date de publication de la demande: 06.09.2000
(73) Titulaire: REXAM SOFAB, 76470 Le Tréport (FR)
(72) Inventeur: BOUGAMONT, Jean-Louis, F-76260 Eu (FR); HENNEMANN, Pascal, F-76260 Eu (FR); LEULIET, David, F-80350 Mers les Bains (FR)
(74) Mandataire: Busnel, Jean-Benoît
(86) Numéro de dépôt international: PCT/FR1998/002520
(87) Numéro de publication internationale: WO 1999/026688

(56) Documents cités:
- EP-A- 0 602 019
- WO-A-94/29187
- FR-A- 2 654 078
- US-A- 4 112 971
- US-A- 4 506 809
- US-A- 4 830 284

## Description

La présente invention concerne un embout pour la distribution de produits pharmaceutiques liquides.

Les embouts traditionnels tels que ceux à application nasale, comprennent généralement un corps cylindroconique destiné à être monté au moyen d'une embase de raccordement sur un réservoir susceptible d'être mis sous pression.

Dans le corps, est ménagé un conduit d'éjection axial renfermant un noyau central et communiquant, d'une part, à son extrémité supérieure avec une valve ou une buse de pulvérisation et, d'autre part, à son extrémité inférieure avec le réservoir via des moyens de pressurisation et/ou de dosage du produit.

De tels embouts sont décrits, par exemple, dans le WO94/29187 où le conduit d'éjection est obturé par une paroi pourvue d'un orifice central coopérant de manière étanche avec un ergot et rattachée à l'embout par des éléments de liaison déformables lui assurant un déplacement axial.

Cependant, avec ces embouts, la protection bactériologique du produit n'est pas assurée de façon fiable.

En effet, la valve ou la buse de pulvérisation ne constitue pas, à elle seule, une barrière mécanique suffisamment efficace pour limiter de façon importante ni a fortiori pour supprimer les risques de contamination du produit.

Bien que l'incorporation d'agents bactéricides ou anti-bactériens dans la matière constitutive des embouts permet d'améliorer la protection, la recherche d'une solution pleinement satisfaisante passe aussi et surtout par la mise au point de moyens mécaniques capables d'assurer une isolation poussée du conduit d'éjection en vue d'obtenir un meilleur confinement du produit dans le réservoir.

Par ailleurs, lorsque le fonctionnement des moyens de pressurisation nécessite un rééquilibrage des pressions entre le réservoir et l'extérieur, la reprise d'air ne doit pas constituer une cause de pollution ou de dégradation du produit et ne doit pas compromettre ni l'étanchéité de l'embout ni la qualité de la distribution ou du dosage.

Enfin, les embouts traditionnels ne présentent pas, en général, une aptitude suffisante à supporter tout mode de stérilisation.

La présente invention a pour but de résoudre ces problèmes techniques de manière satisfaisante.

Ce but est atteint selon l'invention au moyen d'un embout pour la distribution et/ou le dosage de produits pharmaceutiques liquides comprenant un corps cylindroconique muni d'une embase d'appui et de raccordement à un réservoir susceptible d'être mis sous pression et d'un conduit d'éjection axial renfermant un noyau central et communiquant, à une première extrémité, avec une buse de pulvérisation, et, à son autre extrémité, avec le réservoir, le conduit d'éjection étant obturé de manière temporaire et étanche par une paroi qui est solidaire dudit corps et perpendiculaire audit conduit, ladite paroi étant, en outre pourvue d'un orifice central susceptible d'être obturé dans la position de fermeture, par l'appui forcé d'un ergot solidaire du noyau, et rattachée audit corps de façon périphérique par un élément de liaison élastiquement déformable permettant, sous pression, la translation axiale de ladite paroi, caractérisé en ce que ladite paroi est réalisée sous forme d'une membrane dont la perméabilité permet la filtration de l'air.

Selon un mode de réalisation spécifique, la partie supérieure dudit corps est formée d'un capot indépendant portant ladite paroi et coiffant de façon étanche un manchon coaxial solidaire de la partie inférieure dudit corps et délimitant au moins partiellement la partie haute du conduit d'éjection.

Selon une variante, ladite paroi est réalisée d'une seule pièce avec le capot en une matière élastomère ou élastomère-thermoplastique.

Selon une variante particulière, ledit capot est verrouillé sur ledit manchon au moyen d'organes d'encliquetage.

De préférence, ledit manchon se raccorde à la partie inférieure dudit corps par un épaulement transversal formant butée pour le bord inférieur dudit capot.

Selon un autre mode de réalisation, les parties supérieure et inférieure dudit corps sont réalisées en une seule pièce par bi-injection d'une matière élastomère-thermoplastique et d'une matière thermoplastique.

Selon une autre variante, ladite buse de pulvérisation est formée sous ladite paroi par les espaces intercalaires situés radialement à l'extérieur de la périphérie de ladite paroi entre les faces internes de la partie supérieure dudit corps et l'enveloppe externe dudit noyau.

Selon une caractéristique avantageuse, ledit noyau possède une tête élargie sensiblement cylindrique.

Selon encore une autre variante, l'orifice central de ladite paroi est formé d'un alésage cylindrique tandis que ledit ergot a un profil sensiblement tronconique.

De préférence, la face externe de ladite paroi est en retrait par rapport au rebord supérieur dudit corps de façon à définir une cuvette.

Selon encore une autre variante, l'embase inférieure se prolonge vers le bas par une douille destinée à s'emmancher avec serrage radial dans le col du réservoir.

De préférence, ladite paroi est réalisée en silicone.

Selon une variante particulière, ledit noyau assure dans la position de fermeture un état de précontrainte de l'élément de liaison.

L'embout de l'invention offre une étanchéité renforcée du conduit d'éjection interdisant, en position de fermeture, toute pénétration des bactéries ou d'autres contaminants biologiques.

L'utilisation complémentaire d'un agent bactéricide agissant par simple contact avec le produit et sans migration permet alors le traitement aseptique in situ de la fraction de produit restant confinée à l'intérieur de la buse et dans le conduit d'éjection, après retour en position de fermeture de la paroi déplaçable.

De même, ce traitement aseptique affecte aussi les surfaces extérieures de l'embout qui sont plus exposées à des milieux contaminants.

Compte tenu de l'absence de volume mort au niveau de la paroi mobile et des très faibles volumes des espaces intercalaires à l'intérieur de l'embout, le rendement de l'agent bactéricide est excellent et conduit à la neutralisation complète des germes.

Lorsque les moyens de pressurisation utilisés fonctionnent avec une reprise d'air, le volume d'air aspiré est filtré au travers de la paroi déplaçable, ce qui supprime tout risque de pollution et/ou de contamination.

Par ailleurs, l'embout dans son ensemble peut être stérilisé par tout moyen tel que les rayonnements ionisants bêta et gamma et les traitements thermiques à une température d'au moins 120°C

En outre, la pulvérisation est de très bonne qualité et ceci de façon constante et durable du fait que la paroi dans son ensemble se déplace de façon uniforme en translation axiale sans décalage ni déformation parasite.

L'embout de l'invention peut être utilisé de manière avantageuse pour la distribution et le dosage de produits pharmaceutiques à application nasale (sphère ORL) ou ophtalmique.

L'invention sera mieux comprise à la lecture de la description qui va suivre accompagnée du dessin sur lequel :
- la figure 1 représente une vue partielle en coupe d'un mode de réalisation de l'embout de l'invention ;
- les figures 2a, 2b, et 2c représentent des vues partielles en coupe d'un mode de réalisation de l'embout de l'invention, respectivement à l'état libre puis à l'état assemblé en position de fermeture puis en position d'ouverture.

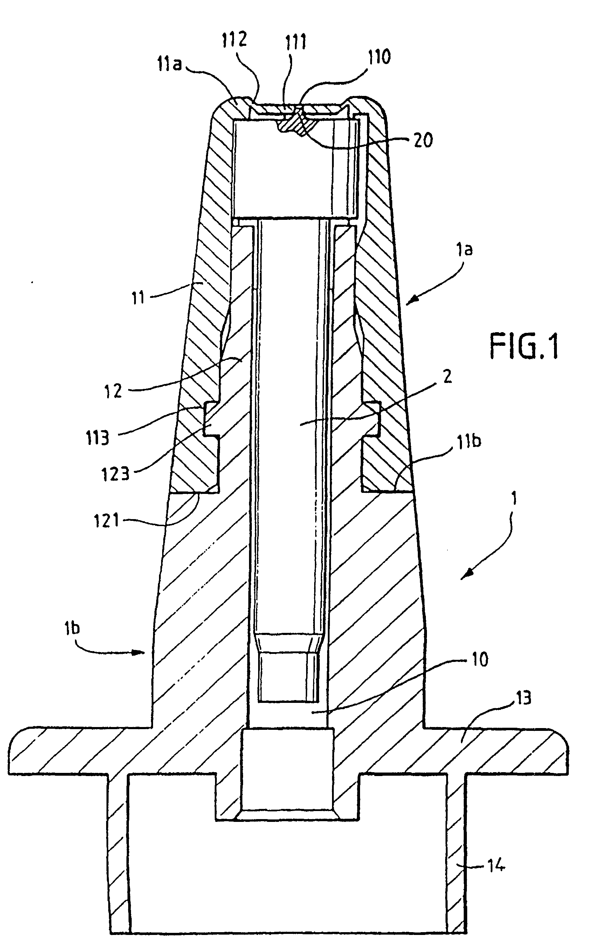

L'embout représenté sur la figure 1 est destiné à délivrer des doses d'un produit pharmaceutique liquide utilisé notamment pour le traitement des affections des voies respiratoires ou pour les traitements ophtalmiques.

Cet embout comprend un corps cylindroconique 1 muni d'une embase inférieure 13 pour l'appui manuel et le raccordement à un réservoir (non représenté). L'embase inférieure 13 est elle-même prolongée vers le bas par une douille 14 destinée à être emmanchée avec serrage radial dans le col cylindrique d'un réservoir.

Dans le corps 1 est ménagé un conduit d'éjection axial 10 renfermant un noyau central 2 réalisé ici sous forme d'une pièce cylindrique pleine et indépendante avec une tête élargie.

Selon une variante, le corps 1 et le noyau sont réalisés en une seule pièce.

Le conduit d'éjection 10 communique, d'une part, à son extrémité supérieure avec une buse de pulvérisation, et d'autre part, à son extrémité inférieure, avec le réservoir, via des moyens de pressurisation et/ou de dosage (non représentés sur la figure 1).

L'extrémité supérieure du conduit d'éjection 10 est obturée de manière temporaire et étanche par une paroi transversale 111. La paroi 111 est déplaçable de façon réversible sous l'action du produit liquide éjecté sous pression.

La paroi 111 est pourvue d'un orifice central 110 formé d'un alésage cylindrique obturé en position de fermeture, par un ergot 20, à profil sensiblement tronconique, solidaire du noyau 2.

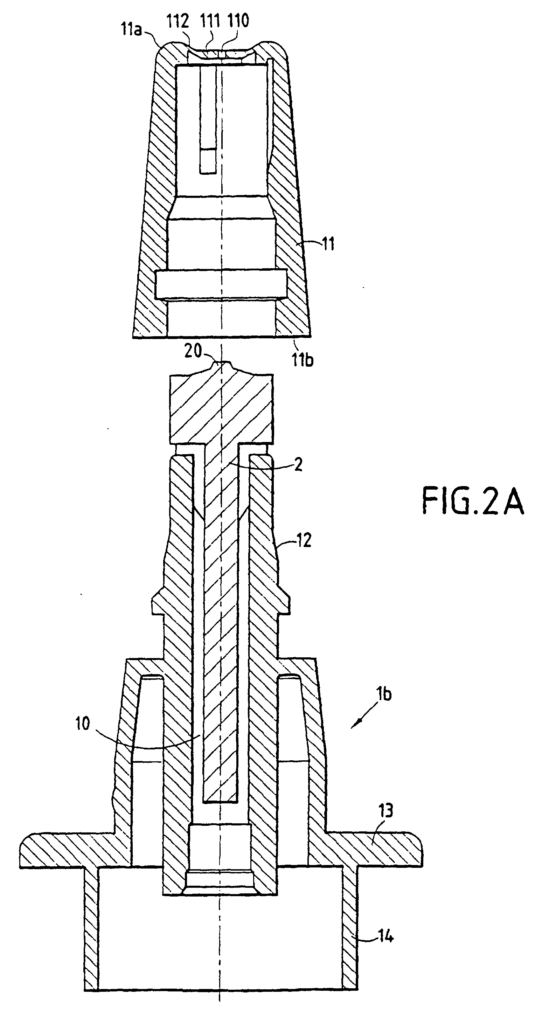

Dans le mode de réalisation de la figure 1, l'ergot 20 vient se loger dans l'alésage 110 tandis que dans le mode de réalisation des figures 2a à 2c, l'ergot 20 vient en appui forcé contre la face interne de la paroi 111 sur et autour de l'alésage 110.

La buse de pulvérisation est de préférence du type micromist (dénomination SOFAB) en étant formée sous la paroi 111 par les espaces intercalaires compris entre les faces internes de la partie supérieure 1a du corps 1 et l'enveloppe externe du noyau 2.

La paroi 111 s'étend perpendiculairement à l'axe du conduit 10 en étant solidaire de la partie supérieure 1a du corps 1.

Le rattachement de la paroi 111 au corps 1 et réalisé de façon périphérique au moyen d'un élément de liaison 112 élastiquement déformable réalisé de préférence sous forme d'un voile annulaire.

L'élément de liaison 112 a une épaisseur plus faible que celle de la paroi 111 qui est donc plus rigide.

Une pression de liquide compris entre 0,1 et 10 bars générée par appui manuel sur l'embase 13 (et actionnement des moyens de pressurisation) est appliquée contre la face interne de la paroi 111 et force l'élément de liaison 112 à se déformer élastiquement en formant ainsi une articulation pour la paroi 111.

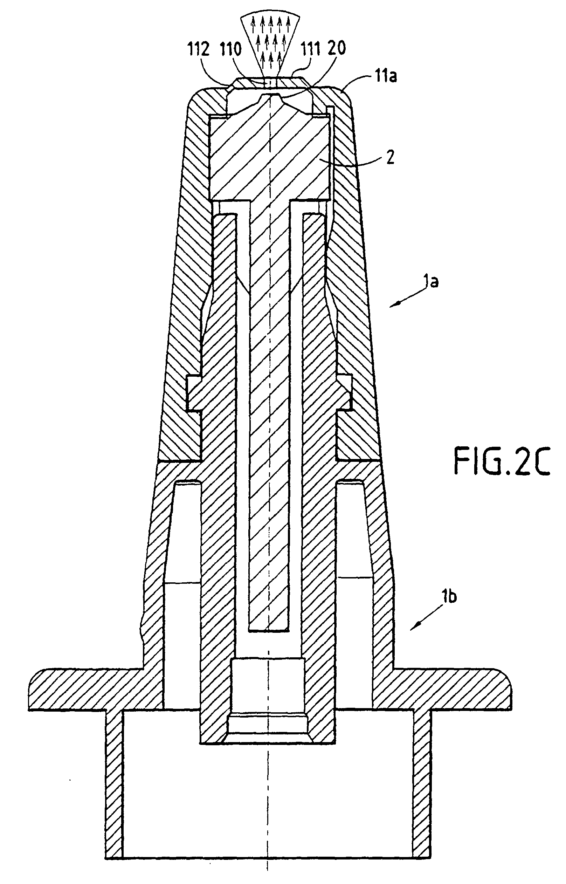

La paroi 111 se déporte alors vers le haut par une translation axiale en se maintenant dans des plans sensiblement parallèles et en libérant l'orifice 110 de l'ergot 20 (voir figure 2c). Lorsque la pression de liquide baisse, l'élément de liaison 112 exerce une force de rappel de la paroi 111 vers sa position de repos qui correspond à l'obturation étanche de l'orifice 110.

Dans la position de fermeture du mode de réalisation de la figure 1 ou bien.

A l'état libre, dans la variante de la figure 2a, le plan de la face externe de la paroi 111 est en retrait par rapport au rebord supérieur curviligne 11a du corps 1 de façon à définir une cuvette.

Cette disposition implique que l'élément de liaison 112 soit incliné vers l'axe du conduit 10 et vers le bas avec un angle compris entre 0 et 60° tandis que la face latérale de la paroi 111 est biseautée en s'étendant dans le prolongement de l'élément de liaison 112.

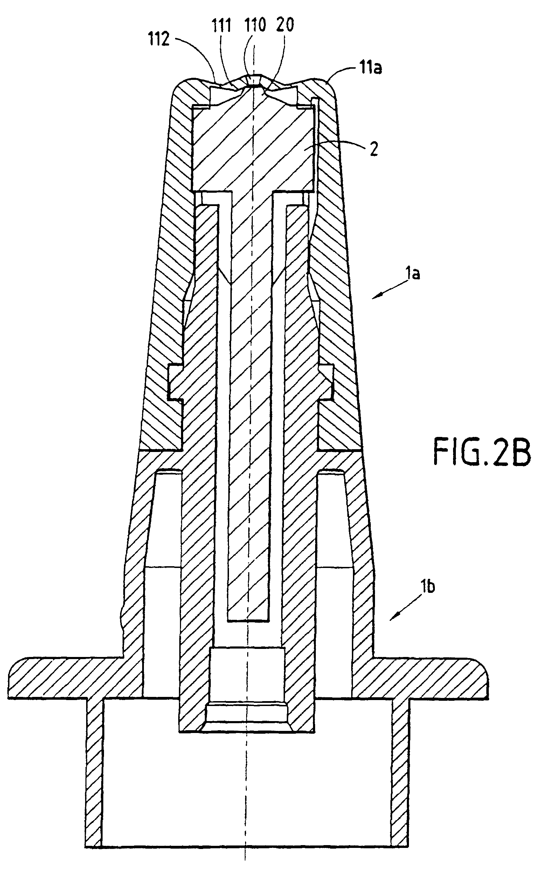

Selon une variante de réalisation représentée sur la figure 2b, à l'état assemblé et en position de fermeture de l'embout, il existe un appui forcé de l'ergot 20 contre la paroi 111, ce qui garantit l'herméticité du conduit 10 et qui entraîne un léger déplacement de la paroi 111 vers l'extérieur.

Selon cette variante, dans la position de fermeture, l'élément de liaison 112 est donc déformé et reste sous contraintes tandis que le profil cylindrique de l'alésage 110 devient sensiblement tronconique.

Bien entendu, la maîtrise de la déformation de l'élément de liaison 112 passe par l'ajustement de ses caractéristiques géométriques et de son module d'élasticité.

Ainsi la pression d'ouverture du conduit 10 dépendra de l'aire de la face interne de la paroi 111 contre laquelle s'exerce la pression du liquide et de la force de rappel de l'élément de liaison 112 qu'il faut vaincre pour libérer l'orifice 110. Globalement, depuis l'état libre (figure 2a) jusqu'à la position d'ouverture de l'état assemblé (figure 2c) la paroi 111 subit bien une translation axiale en passant par un état de précontrainte.

Par ailleurs, le régime de fonctionnement des canaux et du réseau tourbillonnaire de la buse de pulvérisation n'est pas perturbé par le déplacement de la paroi 111 car ils sont disposés radialement à l'extérieur de la périphérie de l'élément de liaison 112.

Dans le mode de réalisation particulier représenté, la partie supérieure 1a du corps 1 est formée d'un capot 11 indépendant dont le bord supérieur 11a porte la paroi 111. Le capot 11 coiffe de façon étanche un manchon coaxial 12 solidaire de la partie inférieure 1b du corps 1. Le manchon 12 délimite, au moins partiellement, la partie haute du conduit d'éjection 10.

La paroi 111 est alors réalisée d'une seule pièce avec le capot 11 en une matière élastomère telle qu'un EPDM (éthylène - propylène - diène - monomère) ou un élastomère-thermoplastique tel qu'un mélange polypropylène-EPDM.

Selon une variante de réalisation, la paroi 111 est réalisée sous forme d'une membrane dont la perméabilité est déterminée de façon à assurer la filtration de l'air entrant pour éviter toute pollution ou contamination (notamment bactérienne) du produit suite à la reprise d'air.

De préférence, le matériau utilisé pour réaliser cette membrane sera du silicone ou un composé élastomère silicone.

La perméabilité de l'air du capot 11 réalisé par moulage à partir d'un élastomère silicone est comprise entre 70µl/24 heures à 800µl/24 heures

Le capot qui est éventuellement amovible est en serrage radial avec la partie haute du manchon 12 et est verrouillé sur la partie basse, par exemple, au moyen d'organes d'encliquetage complémentaires 113,123.

La partie inférieure 1b du corps 1 est réalisée, de préférence en polypropylène ou tout autre matériau susceptible de supporter un traitement thermique de stérilisation.

Le manchon 12 se raccorde à la partie inférieure 2b du corps 1 par un épaulement transversal 121 formant butée pour le bord inférieur 11b du capot

Selon un autre mode de réalisation non représenté, les parties supérieure la et inférieure 1b du corps 1 seront réalisées en une seule pièce par bi-injection dans un moule d'une matière élastomère et d'une matière thermoplastique.

De préférence, toutes les zones et composants de l'embout seront réalisés avec des matières dans lesquelles est incorporé un agent antiseptique (bactéricide ou antibactérien), par exemple à base d'ions argent.

## Revendications

1. Embout pour la distribution et/ou le dosage de produits pharmaceutiques liquides comprenant un corps (1) cylindroconique muni d'une embase (13) d'appui et de raccordement à un réservoir susceptible d'être mis sous pression et d'un conduit d'éjection axial (10) renfermant un noyau central (2) et communiquant, à une première extrémité, avec une buse de pulvérisation, et, à son autre extrémité, avec le réservoir, le conduit d'éjection (10) étant obturé de manière temporaire et étanche par une paroi (111) qui est solidaire dudit corps (1) et perpendiculaire audit conduit, ladite paroi étant, en outre0 pourvue d'un orifice central (110) susceptible d'être obturé dans la position de fermeture, par l'appui forcé d'un ergot (20) solidaire du noyau (2), et rattachée audit corps (1) de façon périphérique par un élément de liaison (112) élastiquement déformable permettant, sous pression, la translation axiale de ladite paroi (111), **caractérisé en ce que** ladite paroi (111) est réalisée sous forme d'une membrane dont la perméabilité permet la filtration de l'air.

2. Embout selon la revendication 1, **caractérisé en ce que** la partie supérieure (1a) dudit corps (1) est formée d'un capot indépendant (11) portant ladite paroi (111) et coiffant de façon étanche un manchon coaxial (12) solidaire dudit corps et délimitant au moins partiellement la partie haute du conduit d'éjection (10).

3. Embout selon la revendication 2, **caractérisé en ce que** ladite paroi (111) est réalisée d'une seule pièce avec le capot (11) en une matière élastomère ou élastomère-thermoplastique.

4. Embout selon la revendication 2 ou 3, **caractérisé en ce que** ledit capot (11) est verrouillé sur ledit manchon (12) au moyen d'organes d'encliquetage (113,123).

5. Embout selon l'une des revendications 2 à 4, **caractérisé en ce que** ledit manchon (12) se raccorde audit corps (1) par un épaulement transversal (121) formant butée pour le bord dudit capot (11).

6. Embout selon l'une des revendications précédentes, **caractérisé en ce que** ledit corps (1) est réalisé en une seule pièce par bi-injection d'une matière élastomère-thermoplastique et d'une matière thermoplastique.

7. Embout selon l'une des revendications précédentes, **caractérisé en ce que** ledit noyau (2) possède une tête élargie sensiblement cylindrique.

8. Embout selon l'une des revendications précédentes, **caractérisé en ce que** l'orifice central de ladite paroi (111) est formé d'un alésage cylindrique tandis que ledit ergot (20) a un profil sensiblement tronconique.

9. Embout selon l'une des revendications précédentes, **caractérisé en ce que** la face externe de ladite paroi (111) est en retrait par rapport au rebord supérieur (11a) dudit corps (1) de façon à définir une cuvette.

10. Embout selon l'une des revendications précédentes, **caractérisé en ce que** ladite buse de pulvérisation est formée sous ladite paroi (111) par les espaces intercalaires situés radialement à l'extérieur de la périphérie de ladite paroi entre les faces internes dudit corps (1) et l'enveloppe externe dudit noyau (2).

11. Embout selon l'une des revendications précédentes, **caractérisé en ce que** l'embase (13) se prolonge vers le bas par une douille (14) destinée à s'emmancher dans le réservoir.

12. Embout selon l'une des revendications précédentes, **caractérisé en ce que** ladite paroi est réalisée en silicone.

13. Embout selon l'une des revendications précédentes, **caractérisé en ce que** ledit noyau (2) assure, dans la position de fermeture, un état de précontrainte de l'élément de liaison (112)

## Patentansprüche

1. Endstück für die Ausgabe und/oder Dosierung von flüssigen pharmazeutischen Produkten, bestehend aus einem kegelzylindrischen Körper (1), der mit einem Sockel (13) zur Abstützung und Verbindung mit einem druckbeaufschlagbaren Behälter sowie mit einem axial verlaufenden und einen zentralen Kern (2) umschließenden Ausstoßkanal (10) versehen ist, der an einem ersten Ende mit einer Sprühdüse und am anderen Ende mit dem Behälter in Verbindung steht, wobei dieser Ausstoßkanal (10) durch eine fest mit dem Körper (1) verbundene und quer zum besagten Ausstoßkanal verlaufende Wand (111) vorübergehend und dicht verschlossen wird und besagte Wand außerdem mit einer zentralen Öffnung (110) versehen ist, die in Schließposition durch zwangsweise Gegenpressung einer mit dem Kern (2) fest verbundenen Nase (20) verschlossen werden kann und rundum mit dem Körper (1) durch ein elastisch verformbares Verbindungselement (112) verbunden ist, das unter Druck eine axiale Bewegung dieser Wand (111) ermöglicht, **dadurch gekennzeichnet, dass** diese Wand (111) in Form einer Membran ausgeführt ist, die aufgrund ihrer Durchlässigkeit eine Luftfiltration zulässt.

2. Endstück nach Anspruch 1, **dadurch gekennzeichnet, dass** das Oberteil (1a) des Körpers (1) in Form einer getrennten Abdeckkappe (11) ausgeführt ist, die besagte Wand (111) trägt und einen koaxial verlaufenden, fest mit besagtem Körper verbundenen und den oberen Teil des Ausstoßkanals (10) zumindest teilweise abgrenzenden Stutzen (12) abdeckt.

3. Endstück nach Anspruch 2, **dadurch gekennzeichnet, dass** die Wand (111) und die Abdeckkappe (11) aus einem Stück aus einem Elastomer oder einem thermoplastischen Elastomer ausgeführt sind.

4. Endstück nach Anspruch 2 oder 3, **dadurch gekennzeichnet, dass** die Abdeckkappe (11) auf dem Stutzen (12) durch Rastelemente (113, 123) verriegelt ist.

5. Endstück nach einem der Ansprüche 2 bis 4, **dadurch gekennzeichnet, dass** sich der Stutzen (12) über eine quer verlaufende und für den Rand der Abdeckkappe (11) einen Anschlag bildende Schulter (121) an besagten Körper (1) anschließt.

6. Endstück nach einem der vorhergehenden Ansprüche, **dadurch gekennzeichnet, dass** der Körper (1) aus einem einzigen durch Zweikomponenten-Spritzgießen eines thermoplastischen Elastomers und eines Thermoplasts hergestellten Stück besteht.

7. Endstück nach einem der vorhergehenden Ansprüche, **dadurch gekennzeichnet, dass** der Kern (2) mit einem im wesentlichen zylinderförmigen verbreiterten Kopf versehen ist.

8. Endstück nach einem der vorhergehenden Ansprüche, **dadurch gekennzeichnet, dass** die zentrale Öffnung der Wand (111) durch eine zylindrische Bohrung gebildet wird, während besagte Nase (20) ein im wesentlichen kegelstumpfartiges Profil aufweist.

9. Endstück nach einem der vorhergehenden Ansprüche, **dadurch gekennzeichnet, dass** die Außenseite der Wand (111) gegenüber dem Oberrand (11a) des Körpers (1) so zurückgesetzt ist, dass sie eine schalenförmige Vertiefung bildet.

10. Endstück nach einem der vorhergehenden Ansprüche, **dadurch gekennzeichnet, dass** die Sprühdüse unter der Wand (111) durch die Zwischenräume gebildet wird, die sich radial außerhalb des Umfangs der besagten Wand zwischen den Innenflächen des Körpers (1) und der Außenhülle des Kerns (2) befinden.

11. Endstück nach einem der vorhergehenden Ansprüche, **dadurch gekennzeichnet, dass** der Sockel (13) nach unten durch eine Hülse (14) verlängert wird, die dazu bestimmt ist, in den Behälter eingeführt zu werden.

12. Endstück nach einem der vorhergehenden Ansprüche, **dadurch gekennzeichnet, dass** besagte Wand aus Silikon ausgeführt ist.

13. Endstück nach einem der vorhergehenden Ansprüche, **dadurch gekennzeichnet, dass** der Kern (2) in Schließstellung die Ausübung einer Vorspannung auf das Verbindungselement (112) gewährleistet.

## Claims

1. An end piece for the distribution and/or dispensing of liquid pharmaceutical products, comprising a cylindroconical body (1) provided with a supporting seat (13) for connection to a reservoir capable of being pressurised and with an axial ejection duct (10) enclosing a central core (2) and communicating, at a first end, with a spray nozzle and, at its other end, with the reservoir, the ejection duct (10) being closed temporarily and in sealed manner by a wall (111) which is integral with said body (1) and perpendicular to said duct, said wall being additionally provided with a central orifice (110) capable of being closed in the closure position by the forced pressure of a lug (20) integral with the core (2) and said wall being connected to said body (1) peripherally by a resiliently deformable connecting element (112) allowing axial translation of said wall (111) under pressure, **characterised in that** said wall (111) takes the form of a membrane whose permeability allows air filtration.

2. An end piece according to claim 1, **characterised in that** the upper part (1a) of said body (1) takes the form of an independent covering cap (11) bearing said wall (111) and covering in sealed manner a coaxial sleeve (12) integral with said body and defining at least partially the top part of the ejection duct (10).

3. An end piece according to claim 2, **characterised in that** said wall (111) is made in one piece with the covering cap (11) from an elastomer or thermoplastic elastomer.

4. An end piece according to claim 2 or claim 3, **characterised in that** said covering cap (11) is locked on said sleeve (12) by means of catch members (113, 123) .

5. An end piece according to any one of claims 2 to 4, **characterised in that** said sleeve (12) is connected to said body (1) by a transverse shoulder (121) forming a limit stop for the edge of said covering cap (11).

6. An end piece according to any one of the preceding claims, **characterised in that** said body (1) is made in a single piece by two-component injection moulding of a thermoplastic elastomer and a thermoplastic.

7. An end piece according to any one of the preceding claims, **characterised in that** said core (2) has a substantially cylindrical widened head.

8. An end piece according to any one of the preceding claims, **characterised in that** the central orifice of said wall (111) takes the form of a cylindrical bore while said lug (20) has a substantially frustoconical profile.

9. An end piece according to any one of the preceding claims, **characterised in that** the outer face of said wall (111) is set back relative to the upper edge (11a) of said body (1) in such a way as to define a dished recess.

10. An end piece according to any one of the preceding claims, **characterised in that** said spray nozzle is formed beneath said wall (111) by interstices situated radially to the outside of the periphery of said wall between the inner faces of said body (1) and the outer sheath of said core (2) .

11. An end piece according to any one of the preceding claims, **characterised in that** the seat (13) is extended downwards by a bush (14) intended for insertion into the reservoir.

12. An end piece according to any one of the preceding claims, **characterised in that** said wall is made of silicone.

13. An end piece according to any one of the preceding claims, **characterised in that**, in the closure position, said core (2) keeps the connecting element (112) in a prestressed state.
